# EUROPEAN PATENT SPECIFICATION

(11) **EP 1 465 541 B1**
(45) Date of publication and mention of the grant of the patent: **12.12.2007**
(21) Application number: 03700269.8
(22) Date of filing: 16.01.2003
(51) Int. Cl.: A61B 19/00, G01B 5/016

(54) **METHOD AND APPARATUS FOR RECONSTRUCTING BONE SURFACES DURING SURGERY**
VERFAHREN UND VORRICHTUNG ZUR REKONSTRUKTION VON KNOCHENOBERFLÄCHEN WÄHREND EINER CHIRURGISCHEN BEHANDLUNG
PROCEDE ET APPAREIL POUR LA RECONSTRUCTION DE SURFACES OSSEUSES PENDANT UNE OPERATION CHIRURGICALE

(30) Priority: 16.01.2002 WO PCT/CA02/00047; 18.01.2002 US 349267 P
(43) Date of publication of application: 13.10.2004
(73) Proprietor: Orthosoft Inc., Montreal, Québec H3C 2N6 (CA)
(72) Inventor: RICHARD, Alain, Montreal, Québec H9J 3S9 (CA)
(74) Representative: Betten & Resch
(86) International application number: PCT/CA2003/000069
(87) International publication number: WO 2003/061501

(56) References cited:
- EP-A- 0 919 203
- WO-A-01/67979
- WO-A-02/36031
- WO-A-99/59106
- WO-A-99/60939
- US-A- 5 564 437
- US-A- 5 871 018
- US-A- 6 006 126
- US-A- 6 033 415

## Description

### FIELD OF THE INVENTION

The invention relates to the field of computer-assisted surgery or image-guided surgery. More specifically, it relates to the reconstruction of the surface of a bone during surgery.

### BACKGROUND OF THE INVENTION

As technology allows us to advance in the field of computer-aided surgery, such systems are becoming more specialized and refined. The advances made for orthopedic surgery are particularly impressive. These systems allow surgeons to prepare for surgery by viewing 3D models of patients' anatomy that were reconstructed using pre-operative images such as scans and x-rays. Virtual planning markers can be inserted into three-dimensional images at any sites of interest and the ideal implant or prosthesis can be designed for a specific patient by constructing virtual implant models and simulating the results with the reconstructed model.

Furthermore, during surgery, many surgical instruments are now tracked and can be displayed on the reconstructed 3D models to provide surgeons with a reference as to where they are within a patient's body. This is a precious asset in surgeries that involve delicate procedures that allow the surgeon very little room to maneuver. Unfortunately, this feature can only be taken advantage of when a 3D reconstruction of the patient's structure has been made. This is done pre-operatively using various imaging technologies and can become quite time-consuming for a surgeon.

However, it is desirable to cut down the pre-operative time a surgeon must spend to prepare a surgery. It is also desirable to develop an application that can use other media than Computer-Tomographic (CT) scans, when these are not available.

Moreover, since it is advantageous to provide a surgeon with visual confirmation of the tasks he is performing during the surgery, there is a need to develop a CT-less intra-operative bone reconstruction system.

EP 0919203 describes a frameless stereotactic tomographic scanner including an imaging device defining a coordinate system in scanner space. A localizer device includes a base portion mounted in a fixed relationship to the imaging device and a free end adapted for selective movement into varied positions near a patient body disposed on the imaging device. A position transducer associated with the localizer device generates, in a localizer space, localizer device tip location information as the localizer device is moved near the patient body. A processor converts the localizer tip location information to converted localizer tip location information in an image space. The imaging device is adapted to generate patient body image information in the image space regarding the patient body disposed on the device. A display unit is included for displaying the patient body image information together with the localizer tip position information on a human readable display monitor. The base portion of the localizer device is adapted for mounting onto the imaging device at a plurality of fixed positions.

US 6,006,126 describes a system for computer graphic determination and display of a patient's anatomy, as from CT or MR scanning, and stored along with associated equipment in an object field including the patient's anatomy. A first digitizing camera structure produces a signal representative of its field-of-view which defines coordinates of index points in its field-of-view. A second digitizing camera structure produces similar output for an offset field-of-view. The two camera positions are defined with respect to the patient's anatomy so that the fields-of-view of the cameras include both the patient's anatomy and the equipment, but are taken from different directions. Index markers are for fixing points in the fields of view and accordingly locate equipment relative to said patient anatomy. The index markers are provided by variety of structures including, light sources in various forms as reflectors, diodes, and laser scanner structures to provide a visible grid, mesh or cloud of points.

US 6,033,415 describes a method for transforming a bone image data set representing at least a partial image of a long bone to a robotic coordinate system, comprising generating the bone image data set from a bone image, registering a bone digitizer arm to the robotic coordinate system, generating a digitized bone data set by taking bone surface position measurements with the digitizer arm, and transforming the bone image data set into the robotic coordinate system by performing a best-fit calculation between coordinates of the bone image data set and corresponding coordinates of the digitized bone data set.

### SUMMARY OF THE INVENTION

Accordingly, an object of the present invention is to reduce pre-operative time in surgical procedures.

Another object of the present invention is to reduce the time of instrumentation calibration in surgical procedures.

A further object of the present invention is to provide a simple CT-less system to use for simple surgical cases that can be used in combination with a . CT-based system for difficult surgical cases.

According to a first broad aspect of the present invention, there is provided a method for intra-operatively presenting an approximate model of an anatomical structure not being part of a living human or animal body, the method comprising: applying a registration tool having a position sensing system associated therewith to a plurality of locations on the anatomical structure; acquiring input data using the registration tool such that a point is registered for each of the locations; processing the input data into an approximate model of the anatomical structure; and displaying the approximate model on an output device.

The registration tool is provided with a tip having flat surface adapted for making contact with the surface of an anatomical structure and registering the normal at the point of contact. Preferably, a cloud of points is displayed as a mosaic on the output device. Alternatively, the cloud of points is smoothed over and a smoothed surface is displayed on the output device. The points at which the data was acquired may also be displayed on the smoothed surface. Also alternatively, a three dimensional reconstruction is done based on the acquired input data.

Additionally, a database of known models of the anatomical surface may be used to attach to the portion of the anatomical surface represented by the cloud of points, the smoothed surface, or the three dimensional reconstruction.

According to a second broad aspect of the present invention, there is provided a system for displaying an approximate model of a surface of an anatomical structure as defined in claim 22.

Preferably, the processing module may also perform either a smoothing of a surface or a reconstruction of a three dimensional model. Also, a database of known models may be present to attach to any portion of the anatomical surface represented by the acquired input data in order to display an entire model of the anatomical surface.

Also preferably, the registration tool has a tip adapted for making contact with the surface of an anatomical structure and registering the normal at the point of contact. The normal for each point of contact is comprised in the input data and used in the representation of the anatomical surface.

According to a third broad aspect of the present invention, there is provided a registration tool as defined in claim 19.

The tool is a double ended tool with a first flat surface at the first end and a second flat surface at a second end also adapted to determine the normal at a point of contact. The first flat surface and the second flat surface have different dimensions.

### BRIEF DESCRIPTION OF THE DRAWINGS

These and other features, aspects and advantages of the present invention will become better understood with regard to the following description and accompanying drawings wherein:
FIG. 1 is a flowchart of the method in accordance with the invention;
FIG. 2 shows the mosaic reconstruction of a bone;
FIG. 3 shows the reconstructed bone after smoothing;
FIG. 4 is a diagram of a registration tool with an adaptive tip;
FIG: 5 is a block diagram of the system in accordance with the invention; and
FIG. 6 is a block diagram of a portion of the system of figure 5.

### DETAILED DESCRIPTION OF THE PREFERRED EMBODIMENT

For the purpose of this description, a total knee replacement surgery will be used to demonstrate the invention. However, it can be appreciated that the invention can be used to reconstruct the surface of any anatomical structure in a body.

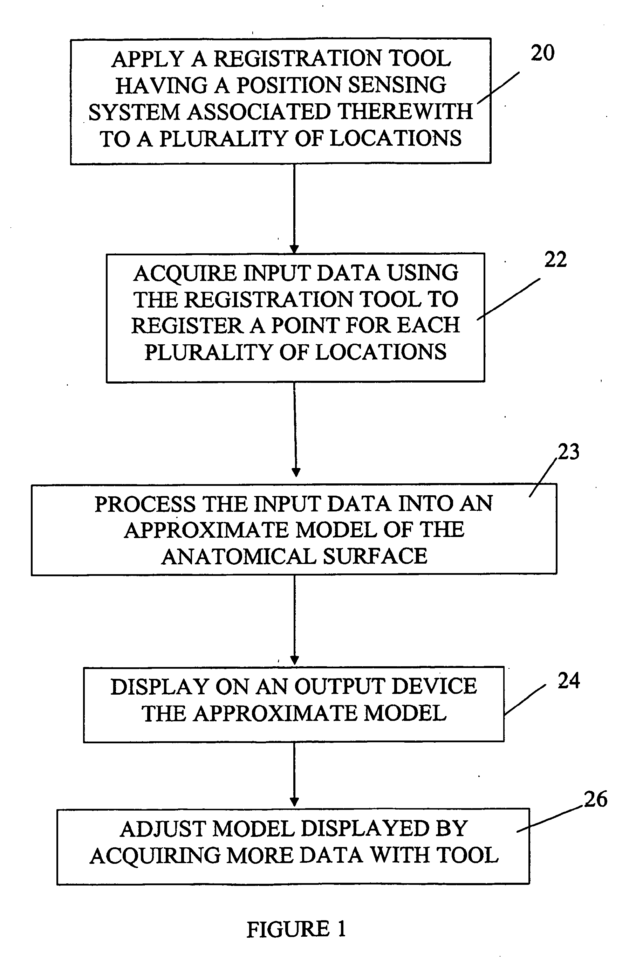

Figure 1 is a flowchart describing the steps used to intra-operatively present an approximate model of an anatomical structure on an output device. The first step is to apply a registration tool to the anatomical surface 20. This tool can be a standard digitizing pointer, a laser pointer, or any other registration tool known to a person skilled in the art. A position sensing system must be associated to the tool to track the position and orientation of the registration tool as it moves over the surface of the anatomical structure. In a preferred embodiment, an infrared, light reflecting tracking system having at least three reflectors is used. Alternatively, any mechanical, electro-magnetic, or optical position sensing system may be used. The next step consists in acquiring input data at each point of contact 22.

In a preferred embodiment, the normal at each point of contact is determined and included in the input data. A tool having a small flat surface, such as a small disc, is used to acquire the data such that instead of registering only a point, a small surface is registered at each point of contact. The input data is then processed into an approximate model of the anatomical surface 23 and is then displayed on the output device 24.

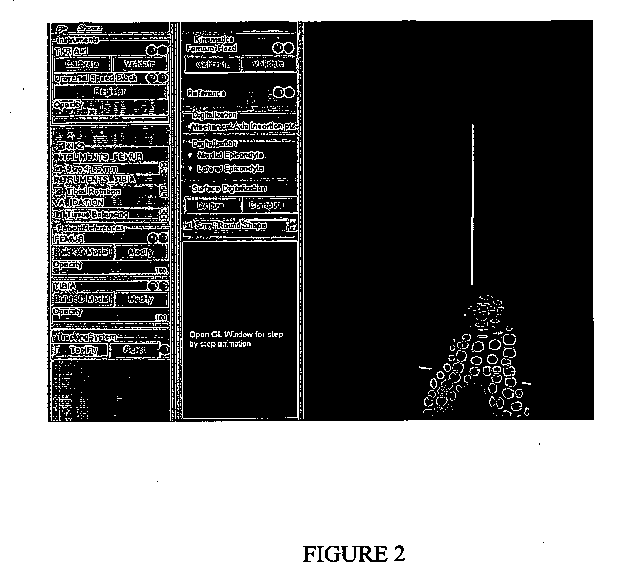

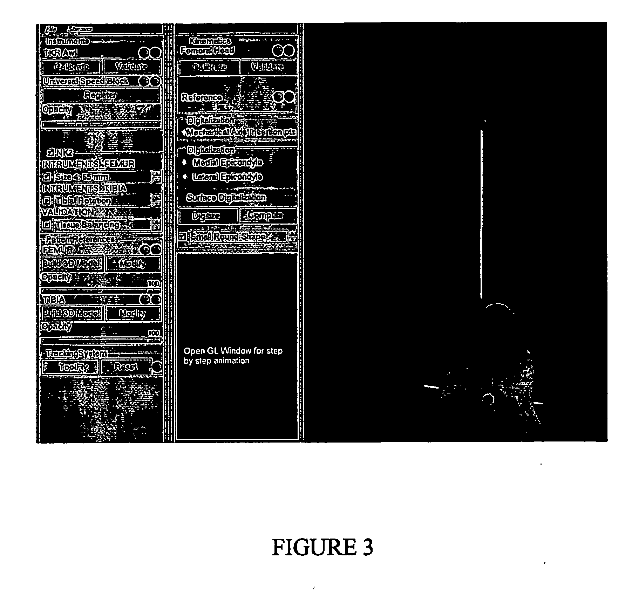

The processing may simply comprise transforming the input data into a cloud of points forming a mosaic representing a portion of the anatomical structure that was digitized. An example of a portion of a femur bone is shown represented by a cloud of points in figure 2. Alternatively, the input data may be smoothed over to be displayed as a smoothed surface representing a more accurate surface topology of the portion of the anatomical structure that was digitized. An example of the same femur bone portion smoothed over can be seen in figure 3. It can be seen from this figure that the normal of each point of contact was taken into consideration when the points were registered. A surface topology is evident from the displayed surface.

The input data may also be used to reconstruct a three dimensional model of the portion of the anatomical structure that was digitized. This requires a more complex processing of the input data than a simple smoothing over. Alternatively, the points registered may be matched to a known model of the same anatomical structure and the model is displayed on the output device with the digitized points indicated on the model. This way, the entire bone can be visualized during the surgery. Alternatively, the input data may be used to reconstruct an entire model of the anatomical surface using extrapolation of the input data.

Another way to display an entire anatomical structure is to attach a portion of a known model to the portion digitized using the registration tool. For example, if the portion of a femur that is digitized consists of the anterior cortex, the condylar surface, and the inter-condylar notch, then a shaft portion and a femoral head from a known model having similar dimensions can be attached to the digitized portion and displayed as an entire femur. The known model can be attached to a cloud of points forming a mosaic, a smoothed surface, or a three dimensional reconstruction.

Optionally, the model of the anatomical structure displayed on the output device may be adjusted by acquiring more points to better represent the actual topology of the anatomical structure. As more data is acquired, the model displayed is updated to reflect the new information.

Once a model representing the anatomy is displayed on the output device, tools used for the surgery can be tracked with respect to this model, thereby allowing the surgeon to navigate with tools and have a reference in the body.

The surface model reconstruction is a process that allows the user to digitize small surfaces instead of points only. These surfaces can be small circles, as can be seen from figure 2. The small circle is physically present on the tip of the registration tool as a small, flat disc. The size of the disc (radius) is chosen as a compromise between accuracy and time. It is counter-productive to ask a surgeon to take hundreds of points when digitizing the surface of a bone. However, the more points taken, the better the representation of the bone and the more accurate the model. The size can also vary depending on the morphology of the bone surface, affecting the precision of the tool. For example, the disc could cover an area of 1cm². The disc must be flat on the surface to register as much surface as possible. The tool also registers the normal at the point of contact between the flat disc surface and the bone. The reconstruction is done in real time.

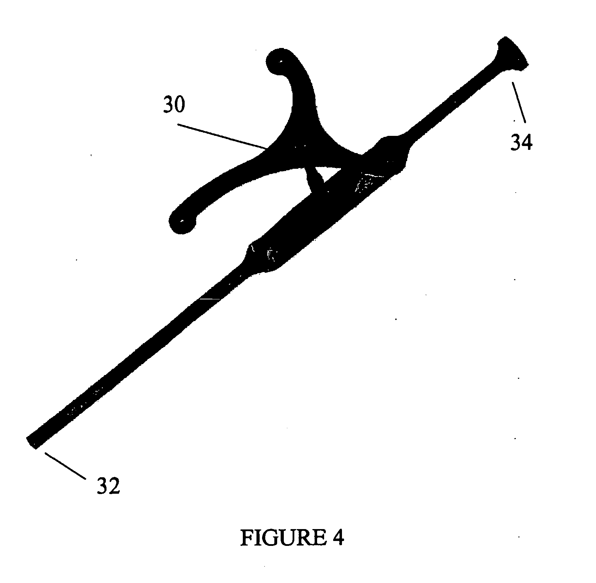

Figure 4 is the preferred embodiment of the registration tool to be used in the digitizing process. The tool is equipped with a position-sensing device 30, such as those known in the field of tracking, having three position identifying devices. In this embodiment, both ends of the tool can serve as a digitizing tip, each end having a different radius. The smaller end 32 can be used on anatomical surfaces that do not easily accommodate the flat surface of the tool. The larger end 34 can be used on flatter anatomical surfaces. The user selects on the computer which end is used. Alternatively, there can be automatic detection of the end being used, such as the computer recognizing the radius of the disc surface when it is placed on the bone surface. For the actual registration of the small surfaces, this can be achieved in several ways. For example, there can be a button on the tool that controls the digitizing. Alternatively, this can be done by pressing a key on a keyboard to select a point to be digitized. Also alternatively, digitizing can be triggered by a rotating action of the tool by a quarter turn. It can be appreciated that alternative embodiments for the registration tool are possible. For example, other multi-purpose combinations can be made. One end can be an awl, a screwdriver, or a probe, while the other end is a digitizer. Similarly, the tool can be a single-ended digitizer as well.

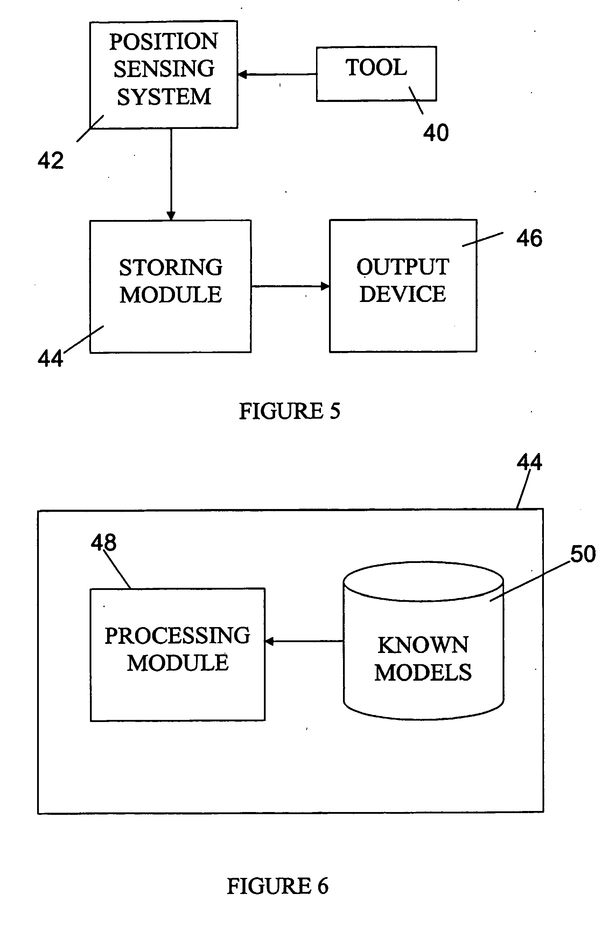

Figure 5 shows the system for displaying an approximate model of a surface of an anatomical structure in accordance with the present invention. A registration tool 40 sends data to a position sensing system 42 corresponding to its position and orientation relative to an anatomical structure. The tool 40 is tracked by the position sensing system 42 in a three-dimensional environment. The orientation and position of the tool 40 is captured by the position sensing system and transferred to a storing module 44. The data is then sent to an output device 46, such as a monitor, to display to the user.

Figure 6 is a block diagram of the storing module 44 in a preferred embodiment. When the data indicating the position and orientation of the tool 40 is received by the storing module 44, it may be processed in various ways. A processing module 48 is used to smooth over the mosaic surface formed by the data recorded by the tool 40. The initial bone registration procedure is done by collecting information on the surface of the bone. The information collected is the position and orientation of the bone surface at each point of contact. The normal of the digitized surface is calculated using the mean value of the orientation of the registration tool 40, which is collected by the sensing system 42. The processing module 48 receives the orientation and position information and uses a surface-modeling algorithm, such as the marching cubes algorithm, to provide a smoothed over surface of the bone topology. It can be appreciated that any surface-modeling algorithm known in the art can be used to perform the smoothing procedure. Optionally, the points at which the initial data was gathered may also be displayed on top of the smoothed surface.

Alternatively, the processing module 48 may perform a three-dimensional reconstruction of a bone using the position and orientation data gathered by the registration tool 40. This reconstruction is similar to a three dimensional reconstruction of a bone done pre-operatively using other types of data gathering devices such as CT-scans and other scanning devices. In one embodiment, the three dimensional reconstruction is done independently of any standard or known shape and size of bone. In a varying embodiment, a database of known models 50 is available to the processing module 48. In this case, the reconstruction is based on known models. The registered points are matched using a best-fit algorithm to a known model of similar size and shape as the anatomical structure under examination. The reconstructed shape is then displayed on the output device 46. The matched points may be displayed on top of the three dimensional shape. In another embodiment, the known models are simply used as a reference for the three dimensional reconstruction. The reconstruction algorithm simply uses the known models as a guide in reconstructing a full three dimensional model.

The known models database 50 comprises a plurality of anatomical structures of varying sizes and shapes. The processing module 48 accesses the database 50 and selects a model of similar size and shape to the anatomical structure undergoing operation. The database 50 may also comprise portions or parts of complete anatomical structures. For example, in the case of a femur bone, the database may comprise femoral heads of different sizes and shapes, or femoral shafts of different sizes and shapes. These parts of anatomical structures are used to attached any one of three dimensional reconstructions, smoothed over surfaces, or clouds of points forming a portion of an anatomical structure. The attached portion provides a more complete visual tool to the surgeon during the surgical procedure. Intra-operative time is saved by limiting the amount of digitizing necessary to have a faithful representation of the areas of interest on the anatomical structure. A better visual tool is provided for guidance during surgical navigation with a computer assisted surgical navigation system.

The above described system may be used independently, or with a complete computer assisted surgical navigation system. Once the intra-operative registration is complete and a representation of the anatomical structure is displayed on the output device, a plurality of surgical tools may be tracked and displayed with respect to the intra-operative representation. Cutting guides and positioning blocks may be tracked and used in conjunction with the displayed representation.

The method and system described above may be used on cadavers or dummies in order to test a computer aided surgery system. Testing of new equipment such as a new tracking system, a positioning block, a cutting guide, or so on can also be done in conjunction with the method and system of the present invention. The method and system described may also be used on cadavers or dummies as a teaching tool for medical students. Real life situations may be simulated using the system in order to practice various surgical procedures without the risks posed to a patient.

It will be understood that numerous modifications thereto will appear to those skilled in the art. Accordingly, the above description and accompanying drawings should be taken as illustrative of the invention and not in a limiting sense. It will further be understood that it is intended to cover any variations, uses, or adaptations of the invention following, in general, the principles of the invention and including such departures from the present disclosure as come within known or customary practice within the art to which the invention pertains and as may be applied to the essential features herein before set forth, and as follows in the scope of the appended claims.

## Claims

1. A method for intra-operatively presenting an approximate model of an anatomical structure, the method comprising:
applying a registration tool (40) having a flat disc surface (32, 34) at a first end and having a position sensing system (30) associated therewith to a plurality of locations on said anatomical structure;
acquiring input data using said registration tool (40) such that a point and its corresponding normal is registered for each of said locations;
processing said input data into an approximate model of said anatomical structure; and
displaying said approximate model on an output device, said anatomical structure not being part of a living human or animal body.

2. A method as claimed in claim 1, wherein said processing comprises processing said input data into a cloud of points forming a mosaic and representing a portion of said anatomical structure.

3. A method as claimed in claim 1, wherein said processing comprises smoothing over a surface represented by said input data to display a smoothed surface of a portion of said anatomical structure.

4. A method as claimed in claim 3, wherein said processing comprises providing on said smoothed surface said plurality of locations where said input data was acquired.

5. A method as claimed in claim 4, comprising repeating said acquiring input data after said displaying to adjust said model of said anatomical structure.

6. A method as claimed in claim 1, wherein said processing comprises reconstructing a three dimensional model using said input data to display a three dimensional model of a portion of said anatomical structure.

7. A method as claimed in claim 1, wherein said processing comprises reconstructing a three dimensional model using said input data and a known model of said anatomical structure to display a three dimensional model of said anatomical structure.

8. A method as claimed in claim 1, wherein said processing comprises selecting a known model from a known model database (50) comprising a plurality of known models of varying sizes and shapes and performing an algorithm to determine a best-fit match of said input data onto said known model.

9. A method as claimed in claim 8, wherein said processing comprises providing on said known model said best-fit match such that said best-fit match is displayed on said output device (46).

10. A method as claimed in claim 2, wherein said processing comprises attaching a portion of a known model of said anatomical structure to said mosaic representing a portion of said anatomical structure, said known model representing a remaining portion of said anatomical structure such that an entire model of said anatomical structure is displayed.

11. A method as claimed in claim 3, wherein said processing comprises attaching a portion of a known model of said anatomical structure to said smoothed surface of a portion of said anatomical structure, said known model representing a remaining portion of said anatomical structure such that an entire model of said anatomical structure is displayed.

12. A method as claimed in claim 6, wherein said processing comprises attaching a portion of a known model of said anatomical structure to said three dimensional model of a portion of said anatomical structure, said known model representing a remaining portion of said anatomical structure such that an entire model of said anatomical structure is displayed.

13. A method as claimed in claim 1, wherein said applying a registration tool (40) comprises applying a registration tool having a flat disc surface at a second end, said flat disc surface at a second end having different dimensions than said flat disc surface at said first end.

14. A method as claimed in claim 1, wherein said applying a registration tool (40) comprises applying a registration tool having an intra-operative tool at a second end.

15. A method as claimed in claim 13, wherein said applying a registration tool comprises selecting one of said first end and said second end to apply to said anatomical surface.

16. A method as claimed in any one of claims 1 and 12, wherein said acquiring input data comprises acquiring data by rotating said tool (40) to indicate to said position sensing system (30) location has been selected.

17. A method as claimed in claim 1, wherein said acquiring input data comprises acquiring data by pressing a switch to indicate to said position sensing system (30) a location has been selected.

18. A method as claimed in any one of claims 1 to 17, wherein said anatomical structure is a bone from one of a cadaver and a dummy.

19. A registration tool (40) for intra-operatively acquiring data representing an approximate model of an anatomical structure and for generating Input data representing a point and its corresponding normal in order to determine a surface topology of said anatomical structure and reconstruct in real-time said approximate model of an anatomical surface; the tool (40) having an adapted tip (34) at a first and such that an anatomical surface and a normal to said anatomical surface are registered when said tip is applied to said anatomical structure, and wherein said adapted tip (34) is a flat disc, wherein a second flat disc (32) is present on a second end of said tool (40), said second flat disc (32) having a radius smaller than said flat disc at said first end (34).

20. A registration tool as claimed in claim 19, wherein said flat disc (34) has a surface area of 1 cm².

21. A registration tool as claimed in claim 19, wherein a rotation of said tool (40) causes data to be acquired.

22. A system for displaying an approximate model of a surface of an anatomical structure, the system comprising:
a registration tool for intra-operatively acquiring data representing an approximate model of an anatomical structure and for generating input data representing a point and its corresponding normal in order to determine a surface topology of said anatomical structure and reconstruct in real-time said approximate model of an anatomical surface; the tool (40) having an adapted tip (34) at a first end such that an anatomical surface and a normal to said anatomical surface are registered when said tip is applied to said anatomical structure, and wherein said adapted tip (34) is a flat disc,
a position sensing system (30) associated to said registration tool (40) for acquiring input data representing a plurality of locations on said surface of an anatomical structure such that a position and orientation of said registration tool (40) is determined at each of said plurality of locations;
a storing module (44) for receiving and storing said input data from said position sensing system (30);
a processing module (48) for processing said input data into an approximate model of said anatomical structure while taking into account said corresponding normal in order to determine a surface topology of said anatomical structure and reconstruct in real-time an intra-operative representation of said anatomical surface; and
an output device (46) for displaying said approximate model of said anatomical structure.

23. A system as claimed in claim 22, wherein said processing module processes said input data into a cloud of points forming a mosaic and representing a portion of said anatomical structure.

24. A system as claimed in claim 22, wherein said processing module smoothes over a surface represented by said input data into a smoothed surface of a portion of said anatomical structure.

25. A system as claimed in claim 24, wherein said processing module displays on top of said smoothed surface of said locations on said anatomical surface where data was acquired.

26. A system as claimed in claim 22, wherein said processing module reconstructs a three dimensional model, using said input data, into a three dimensional model of a portion of said anatomical structure.

27. A system as claimed in any one of claims 23 to 26, comprising a database (50) of known models of varying dimensions, and wherein said processing module (48) attaches a portion of said known models to a portion of said anatomical structure in order to display an entire model.

## Patentansprüche

1. Verfahren zum intraoperativen Darstellen eines Näherungsmodells einer anatomischen Struktur, wobei das Verfahren umfasst:
Anbringen eines Aufzeichnungswerkzeugs (40), das an einem ersten Ende eine flache Scheibenoberfläche (32, 34) besitzt und ein ihm zugeordnetes Positionserfassungssystem (30) besitzt, an mehreren Orten an der anatomischen Struktur;
Erfassen von Eingangsdaten unter Verwendung des Aufzeichnungswerkzeugs (40), derart, dass ein Punkt und seine entsprechende Normale für jeden der Orte aufgezeichnet werden;
Verarbeiten der Eingangsdaten in ein Näherungsmodell der anatomischen Struktur; und
Anzeigen des Näherungsmodells auf einer Ausgabevorrichtung,
wobei die anatomische Struktur kein Teil eines lebenden menschlichen oder tierischen Körpers ist.

2. Verfahren nach Anspruch 1, wobei das Verarbeiten das Verarbeiten der Eingangsdaten in eine Wolke von Punkten, die ein Mosaik bilden und einen Abschnitt der anatomischen Struktur darstellen, umfasst.

3. Verfahren nach Anspruch 1, wobei das Verarbeiten das Glätten über eine Oberfläche, die durch die Eingangsdaten dargestellt wird, umfasst, um eine geglättete Oberfläche eines Abschnitts der anatomischen Struktur anzuzeigen.

4. Verfahren nach Anspruch 3, wobei das Verarbeiten das Vorsehen der mehreren Orte, an denen die Eingangsdaten erfasst wurden, auf der geglätteten Oberfläche umfasst.

5. Verfahren nach Anspruch 4, das das Wiederholen des Erfassens von Eingangsdaten nach dem Anzeigen umfasst, um das Modell der anatomischen Struktur einzustellen.

6. Verfahren nach Anspruch 1, wobei das Verarbeiten das Rekonstruieren eines dreidimensionalen Modells unter Verwendung der Eingangsdaten umfasst, um ein dreidimensionales Modell eines Abschnitts der anatomischen Struktur anzuzeigen.

7. Verfahren nach Anspruch 1, wobei das Verarbeiten das Rekonstruieren eines dreidimensionalen Modells unter Verwendung der Eingangsdaten und eines bekannten Modells der anatomischen Struktur umfasst, um ein dreidimensionales Modell der anatomischen Struktur anzuzeigen.

8. Verfahren nach Anspruch 1, wobei das Verarbeiten das Auswählen eines bekannten Modells aus einer Datenbank (50) für bekannte Modelle, die mehrere bekannte Modelle mit unterschiedlichen Größen und Formen enthält, und das Ausführen eines Algorithmus, um eine am besten passende Anpassung der Eingangsdaten mit dem bekannten Modell zu bestimmen, umfasst.

9. Verfahren nach Anspruch 8, wobei das Verarbeiten das Vorsehen der am besten passenden Anpassung an dem bekannten Modell, derart, dass die am besten passende Anpassung auf der Ausgabevorrichtung (46) angezeigt wird, umfasst.

10. Verfahren nach Anspruch 2, wobei das Verarbeiten das Befestigen eines Abschnitts eines bekannten Modells der anatomischen Struktur an dem einen Abschnitt der anatomischen Struktur darstellenden Mosaik umfasst, wobei das bekannte Modell einen verbleibenden Abschnitt der anatomischen Struktur darstellt, so dass ein gesamtes Modell der anatomischen Struktur angezeigt wird.

11. Verfahren nach Anspruch 3, wobei das Verarbeiten das Befestigen eines Abschnitts eines bekannten Modells der anatomischen Struktur an der geglätteten Oberfläche eines Abschnitts der anatomischen Struktur umfasst, wobei das bekannte Modell einen verbleibenden Abschnitt der anatomischen Struktur darstellt, so dass ein gesamtes Modell der anatomischen Struktur angezeigt wird.

12. Verfahren nach Anspruch 6, wobei das Verarbeiten das Befestigen eines Abschnitts eines bekannten Modells der anatomischen Struktur an dem dreidimensionalen Modell eines Abschnitts der anatomischen Struktur umfasst, wobei das bekannte Modell einen verbleibenden Abschnitt der anatomischen Struktur darstellt, so dass ein gesamtes Modell der anatomischen Struktur angezeigt wird.

13. Verfahren nach Anspruch 1, wobei das Anbringen eines Aufzeichnungswerkzeugs (40) das Anbringen eines Aufzeichnungswerkzeugs mit einer flachen Scheibenoberfläche an einem zweiten Ende umfasst, wobei die flache Scheibenoberfläche an einem zweiten Ende andere Abmessungen als die flache Scheibenoberfläche an dem ersten Ende besitzt.

14. Verfahren nach Anspruch 1, wobei das Anbringen eines Aufzeichnungswerkzeugs (40) das Anbringen an einem zweiten Ende eines Aufzeichnungswerkzeugs, das ein intraoperatives Werkzeug besitzt, umfasst.

15. Verfahren nach Anspruch 13, wobei das Anbringen eines Aufzeichnungswerkzeugs das Auswählen des ersten oder des zweiten Endes für die Anbringung an der anatomischen Oberfläche umfasst.

16. Verfahren nach einem der Ansprüche 1 und 12, wobei das Erfassen von Eingangsdaten das Erfassen von Daten durch Drehen des Werkzeugs (40) umfasst, um dem Positionserfassungssystem (30) zu melden, dass ein Ort ausgewählt worden ist.

17. Verfahren nach Anspruch 1, wobei das Erfassen von Eingangsdaten das Erfassen von Daten durch Drücken eines Schalters umfasst, um dem Positionserfassungssystem (30) zu melden, dass ein Ort ausgewählt worden ist.

18. Verfahren nach einem der Ansprüche 1 bis 17, wobei die anatomische Struktur ein Knochen eines Kadavers oder einer Attrappe ist.

19. Aufzeichnungswerkzeug (40) zum intraoperativen Erfassen von Daten, die ein Näherungsmodell einer anatomischen Struktur darstellen, und zum Erzeugen von Eingangsdaten, die einen Punkt und seine entsprechende Normale darstellen, um eine Oberflächentopologie der anatomischen Struktur zu bestimmen und um in Echtzeit das Näherungsmodell einer anatomischen Struktur zu rekonstruieren; wobei das Werkzeug (40) an einem ersten Ende einen angepassten Kopf (34) besitzt, derart, dass eine anatomische Oberfläche und eine Normale auf dieser anatomischen Oberfläche aufgezeichnet werden, wenn der Kopf an der anatomischen Struktur angebracht wird, wobei der angepasste Kopf (34) eine flache Scheibe ist, wobei eine zweite flache Scheibe (32) an einem zweiten Ende des Werkzeugs (40) vorhanden ist, wobei die zweite flache Scheibe (32) einen Radius hat, der kleiner als jener der flachen Scheibe an dem ersten Ende (34) ist.

20. Aufzeichnungswerkzeug nach Anspruch 19, wobei die flache Scheibe (34) einen Flächeninhalt von 1 cm² hat.

21. Aufzeichnungswerkzeug nach Anspruch 19, wobei ein Drehen des Werkzeugs (40) bewirkt, dass Daten erfasst werden.

22. System zum Anzeigen eines Näherungsmodells einer Oberfläche einer anatomischen Struktur, wobei das System umfasst:
ein Aufzeichnungswerkzeug zum interaoperativen Erfassen von Daten, die ein Näherungsmodell einer anatomischen Struktur darstellen, und zum Erzeugen von Eingangsdaten, die einen Punkt und seine entsprechende Normale darstellen, um eine Oberflächentopologie der anatomischen Struktur zu bestimmen und um in Echtzeit das Näherungsmodell einer anatomischen Oberfläche zu rekonstruieren; wobei das Werkzeug (40) an einem ersten Ende einen angepassten Kopf (34) besitzt, derart, dass eine anatomische Oberfläche und eine Normale auf dieser anatomischen Oberfläche aufgezeichnet werden, wenn der Kopf an der anatomischen Struktur angebracht wird, und wobei der angepasste Kopf (34) eine flache Scheibe ist,
ein Positionserfassungssystem (30), das dem Aufzeichnungswerkzeug (40) zugeordnet ist, um Eingangsdaten zu erfassen, die mehrere Orte auf der Oberfläche einer anatomischen Struktur darstellen, so dass eine Position und eine Orientierung des Aufzeichnungswerkzeugs (40) an jedem der mehreren Orte bestimmt werden;
ein Speichermodul (44) zum Empfangen und Speichern der Eingangsdaten von dem Positionserfassungssystem (30);
ein Verarbeitungsmodul (48) zum Verarbeiten der Eingangsdaten in ein Näherungsmodell der anatomischen Struktur, wobei die entsprechende Normale berücksichtigt wird, um eine Oberflächentopologie der anatomischen Struktur zu bestimmen und um in Echtzeit eine intraoperative Darstellung der anatomischen Struktur zu rekonstruieren; und
eine Ausgabevorrichtung (46) zum Anzeigen des Näherungsmodells der anatomischen Struktur.

23. System nach Anspruch 22, wobei das Verarbeitungsmodul die Eingangsdaten in eine Wolke von Punkten verarbeitet, die ein Mosaik bilden und einen Abschnitt der anatomischen Struktur darstellen.

24. System nach Anspruch 22, wobei das Verarbeitungsmodul einen Abschnitt der anatomischen Struktur über eine durch die Eingangsdaten dargestellte Oberfläche in eine geglättete Oberfläche glättet.

25. System nach Anspruch 24, wobei das Verarbeitungsmodul auf der Oberseite der geglätteten Oberfläche die Orte auf der anatomischen Struktur, wo Daten erfasst wurden, anzeigt.

26. System nach Anspruch 22, wobei das Verarbeitungsmodul ein dreidimensionales Modell unter Verwendung der Eingangsdaten in ein dreidimensionales Modell eines Abschnitts der anatomischen Struktur rekonstruiert.

27. System nach einem der Ansprüche 23 bis 26, das eine Datenbank (50) für bekannte Modelle mit unterschiedlichen Abmessungen umfasst, wobei das Verarbeitungsmodul (48) einen Abschnitt der bekannten Modelle an einem Abschnitt der anatomischen Struktur befestigt, um ein gesamtes Modell anzuzeigen.

## Revendications

1. Procédé de présentation intraopératoire d'un modèle approximatif d'une structure anatomique, le procédé comprenant :
l'application d'un outil d'enregistrement (40) ayant une surface de disque plat (32, 34) au niveau d'une première extrémité et ayant un système de détection de position (30) associé à celui-ci à une pluralité d'endroits sur ladite structure anatomique ;
l'acquisition de données d'entrée en utilisant ledit outil d'enregistrement (40) de telle manière qu'un point et sa normale correspondante sont enregistrés pour chacun desdits emplacements ;
le traitement desdites données d'entrée dans un modèle approximatif de ladite structure anatomique ; et
l'affichage dudit modèle approximatif sur un dispositif de sortie,
ladite structure anatomique ne faisant pas partie du corps d'un animal ou d'un être humain vivants.

2. Procédé selon la revendication 1, dans lequel ledit traitement comprend la transformation desdites données d'entrée en un nuage de points formant une mosaïque et représentant une partie de ladite structure anatomique.

3. Procédé selon la revendication 1, dans lequel ledit traitement comprend le lissage d'une surface représentée par lesdites données d'entrée pour afficher une surface lisse d'une partie de ladite structure anatomique.

4. Procédé selon la revendication 3, dans lequel ledit traitement comprend la fourniture sur ladite surface lisse de ladite pluralité d'emplacements où lesdites données d'entrée ont été acquises.

5. Procédé selon la revendication 4, comprenant la répétition de ladite acquisition des données d'entrée après ledit affichage pour ajuster ledit modèle de ladite structure anatomique.

6. Procédé selon la revendication 1, dans lequel ledit traitement comprend la reconstruction d'un modèle en trois dimensions en utilisant lesdites données d'entrée pour afficher un modèle en trois dimensions d'une partie de ladite structure anatomique.

7. Procédé selon la revendication 1, dans lequel ledit traitement comprend la reconstruction d'un modèle en trois dimensions en utilisant lesdites données d'entrée et un modèle connu de ladite structure anatomique pour afficher un modèle en trois dimensions de ladite structure anatomique.

8. Procédé selon la revendication 1, dans lequel ledit traitement comprend le choix d'un modèle connu dans une base de données (50) de modèles connus comprenant une pluralité de modèles connus de différentes tailles et formes et la réalisation d'un algorithme pour déterminer la correspondance la mieux ajustée desdites données d'entrée sur ledit modèle connu.

9. Procédé selon la revendication 8, dans lequel ledit traitement comprend la fourniture sur ledit modèle connu de ladite correspondance la mieux adaptée de telle manière que ladite correspondance la mieux adaptée est affichée sur ledit dispositif de sortie (46).

10. Procédé selon la revendication 2, dans lequel ledit traitement comprend la fixation d'une partie d'un modèle connu de ladite structure anatomique à ladite mosaïque représentant une partie de ladite structure anatomique, ledit modèle connu représentant une partie restante de ladite structure anatomique de telle manière qu'un modèle entier de ladite structure anatomique est affiché.

11. Procédé selon la revendication 3, dans lequel ledit traitement comprend la fixation d'une partie d'un modèle connu de ladite structure anatomique à ladite surface lisse d'une partie de ladite structure anatomique, ledit modèle connu représentant une partie restante de ladite structure anatomique de telle manière qu'un modèle entier de ladite structure anatomique est affiché.

12. Procédé selon la revendication 6, dans lequel ledit traitement comprend la fixation d'une partie d'un modèle connu de ladite structure anatomique au dit modèle en trois dimensions d'une partie de ladite structure anatomique, ledit modèle connu représentant une partie restante de ladite structure anatomique de telle manière qu'un modèle entier de ladite structure anatomique est affiché.

13. Procédé selon la revendication 1, dans lequel ladite application d'un outil d'enregistrement (40) comprend l'application d'un outil d'enregistrement ayant une surface de disque plat au niveau d'une seconde extrémité, ladite surface de disque plat au niveau d'une seconde extrémité ayant des dimensions différentes de celles de ladite surface de disque plat au niveau de ladite première extrémité.

14. Procédé selon la revendication 1, dans lequel ladite application d'un outil d'enregistrement (40) comprend l'application d'un outil d'enregistrement ayant un outil intraopératoire au niveau d'une seconde extrémité.

15. Procédé selon la revendication 13, dans lequel ladite application d'un outil d'enregistrement comprend le choix de l'une de ladite première extrémité et de ladite seconde extrémité pour appliquer à ladite surface anatomique.

16. Procédé selon l'une quelconque des revendications 1 et 12, dans lequel ladite acquisition de données d'entrée comprend l'acquisition de données par rotation dudit outil (40) pour indiquer au dit système de détection de position (30) qu'un emplacement a été choisi.

17. Procédé selon la revendication 1, dans lequel ladite acquisition de données d'entrée comprend l'acquisition de données en appuyant sur un bouton pour indiquer au dit système de détection de position (30) qu'un emplacement a été choisi.

18. Procédé selon l'une quelconque des revendications 1 à 17, dans lequel ladite structure anatomique est un os de l'un d'un cadavre et d'un mannequin.

19. Outil d'enregistrement (40) destiné à acquérir de manière intraopératoire des données représentant un modèle approximatif d'une structure anatomique et à générer des données d'entrée représentant un point et sa normale correspondante de manière à déterminer une topologie de surface de ladite structure anatomique et à reconstruire en temps réel ledit modèle approximatif d'une surface anatomique ; l'outil (40) ayant une pointe adaptée (34) au niveau d'une première extrémité de telle manière qu'une surface anatomique et une normale à ladite surface anatomique sont enregistrées lorsque ladite pointe est appliquée à ladite structure anatomique, et dans lequel ladite pointe adaptée (34) est un disque plat, dans lequel un second disque plat (32) est présent sur une seconde extrémité dudit outil (40), ledit second disque plat (32) ayant un rayon inférieur au dit disque plat au niveau de ladite première extrémité (34).

20. Outil d'enregistrement selon la revendication 19, dans lequel ledit disque plat (34) a une aire de surface de 1 cm².

21. Outil d'enregistrement selon la revendication 19, dans lequel une rotation dudit outil (40) entraîne l'acquisition des données.

22. Système d'affichage d'un modèle approximatif d'une surface d'une structure anatomique, le système comprenant :
un outil d'enregistrement destiné à acquérir de manière intraopératoire des données représentant un modèle approximatif d'une structure anatomique et à générer des données d'entrée représentant un point et sa normale correspondante de manière à déterminer une topologie de surface de ladite structure anatomique et à reconstruire en temps réel ledit modèle approximatif d'une structure anatomique ; l'outil (40) ayant une pointe adaptée (34) au niveau d'une première extrémité de telle manière qu'une surface anatomique et une normale à ladite surface anatomique sont enregistrées lorsque ladite pointe est appliquée à ladite structure anatomique, et dans lequel ladite pointe adaptée (34) est un disque plat,
un système de détection de position (30) associé au dit outil d'enregistrement (40) pour l'acquisition de données d'entrée représentant une pluralité d'emplacements sur ladite surface d'une structure anatomique, de telle manière qu'une position et une orientation dudit outil d'enregistrement (40) sont déterminées au niveau de chacun des emplacements de ladite pluralité d'emplacements;
un module de stockage (44) destiné à recevoir et à stocker lesdites données d'entrée dudit système de détection de position (30);
un module de traitement (48) destiné à traiter lesdites données d'entrée en un modèle approximatif de ladite structure anatomique tout en prenant en considération ladite normale correspondante de manière à déterminer une topologie de surface de ladite structure anatomique et à reconstruire en temps réel une représentation intraopératoire de ladite surface anatomique ; et
un dispositif de sortie (46) pour afficher ledit modèle approximatif de ladite structure anatomique.

23. Système selon la revendication 22, dans lequel ledit module de traitement transforme lesdites données d'entrée en un nuage de points formant une mosaïque et représentant une partie de ladite structure anatomique.

24. Système selon la revendication 22, dans lequel ledit module de traitement lisse une surface représentée par lesdites données d'entrée en une surface lisse d'une partie de ladite structure anatomique.

25. Système selon la revendication 24, dans lequel ledit module de traitement affiche sur le dessus de ladite surface lissée desdits emplacements sur ladite surface anatomique où les données ont été acquises.

26. Système selon la revendication 22, dans lequel ledit module de traitement reconstruit un modèle en trois dimensions, en utilisant lesdites données d'entrée, en un modèle en trois dimensions d'une partie de ladite structure anatomique.

27. Système selon l'une quelconque des revendications 23 à 26, comprenant une base de données (50) de modèles connus de différentes dimensions, et dans lequel ledit module de traitement (48) fixe une partie desdits modèles connus à une partie de ladite structure anatomique de manière à afficher un modèle entier.
